# EUROPEAN PATENT APPLICATION

(11) **EP 1 338 608 A2**
(43) Date of publication of application: **27.08.2003**
(21) Application number: 02102902.0
(22) Date of filing: 20.12.2002
(51) Int. Cl.: C07K 14/39, C07K 14/47, C12N 15/11, C12N 15/62

(54) **Protein complexes and methods for their use**

(30) Priority: 20.12.2001 EP 01130253
(71) Applicant: CELLZOME AG, 69117 Heidelberg (DE)
(72) Inventor: Bauer, Andreas, Dr., 69123 Heidelberg (DE); Gavin, Anne-Claude, Dr., 69181 Heidelberg (DE); Superti-Furga, Gulio, Dr., 69118 Heidelberg (DE); Küster, Bernhard, Dr., 69254 Malsch (DE); Schultz, Jörg, Dr., 69126 Heidelberg (DE); Marzioch, Martina, Dr., 69126 Heidelberg (DE); Grandi, Paola, Dr., 69126 Heidelberg (DE); Krause, Roland, 69115 Heidelberg (DE); Kruse, Ulrich, Dr., 69221 Dossenheim (DE); Merino, Alejandro, Dr., 69117 Heidelberg (DE); Bauch, Angela, Dr., 69117 Heidelberg (DE); Michon, Anne-Marie, Dr., 69118 Heidelberg (DE); Leutwein, Christina, Dr., 69115 Heidelberg (DE); Rick, Jens, 69115 Heidelberg (DE)
(74) Representative: Grünecker, Kinkeldey, Stockmair & Schwanhäusser Anwaltssozietät

(57) **Abstract**

The present invention relates to multiprotein complexes from eukaryotes. The complexes are obtainable by using a protein as a bait and isolating the set of proteins which is attached thereto from cells. Such protein complexes may comprise up to 30 distinct proteins.

## Description

With reference to the decision of the President of the European Patent Office dated 9 June 2000, the full version of this document will be published in electronic form only.

Conformément à la décision du Président de l'Office européen des brevets, en date du 9 juin 2000, le document complet sera publié que sous forme électronique.

Mit Bezug auf den Beschluß des Präsidenten des Europäischen Patentamts vom 9. Juni 2000, wird die vollständige Fassung dieses Dokuments nur elektronisch veröffentlich.

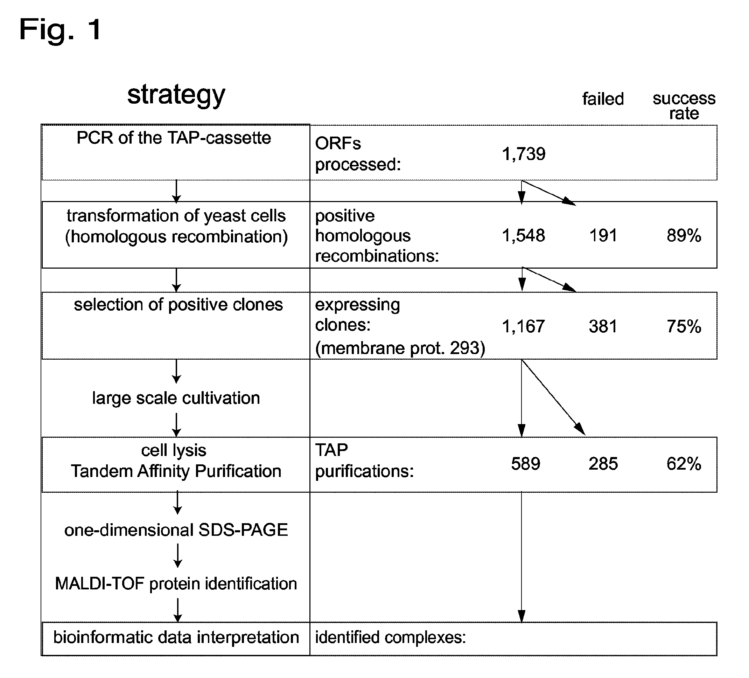

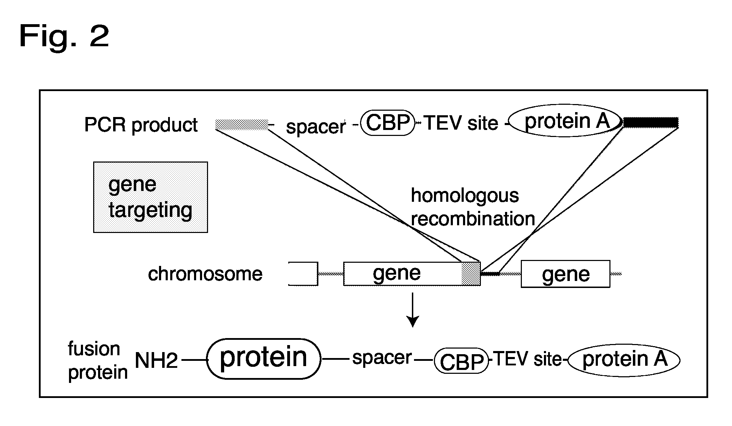

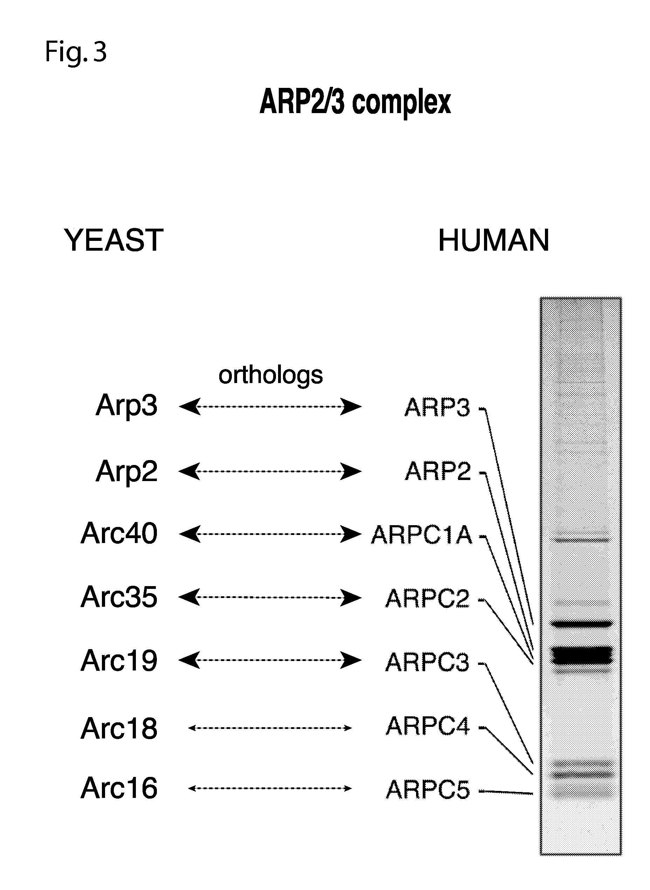

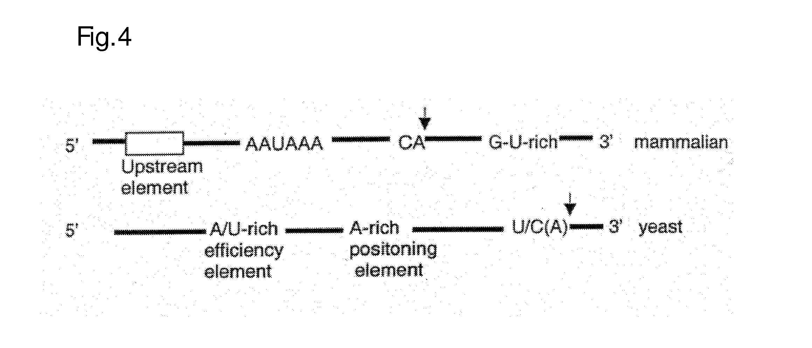

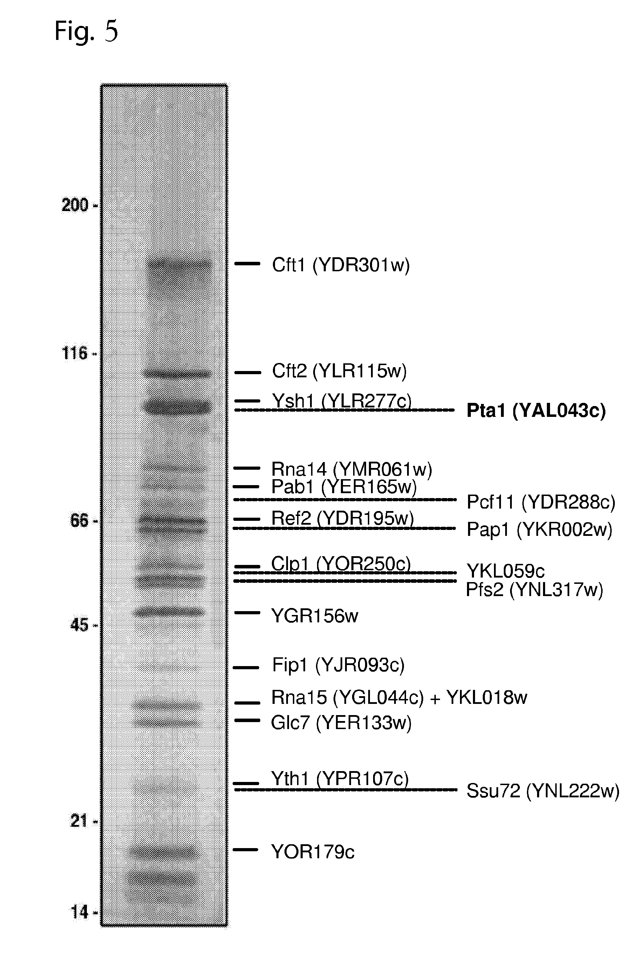

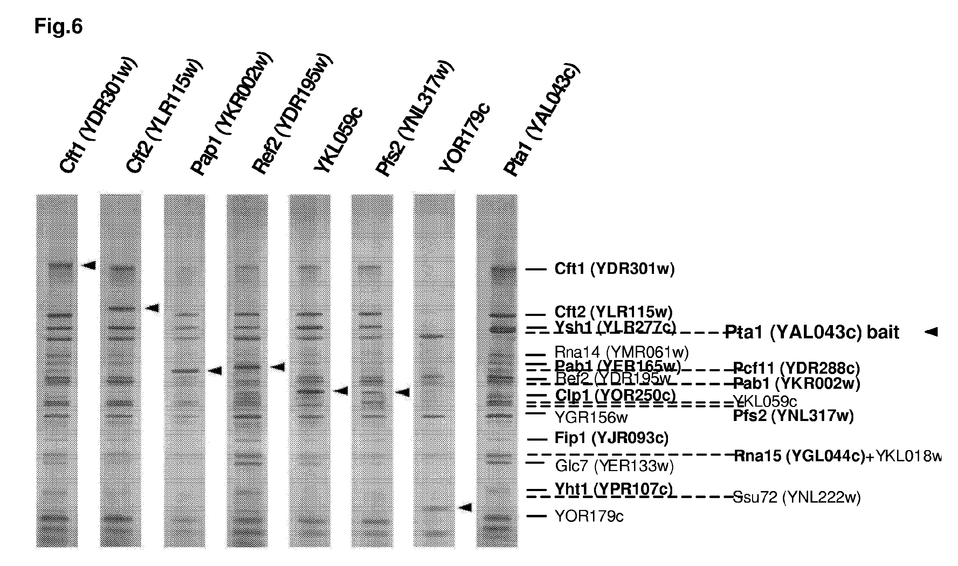

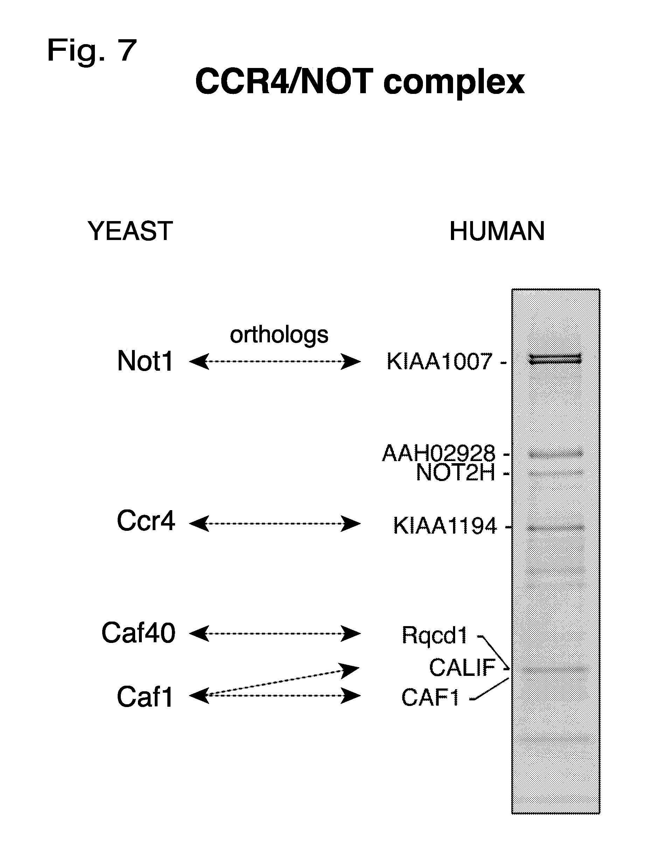

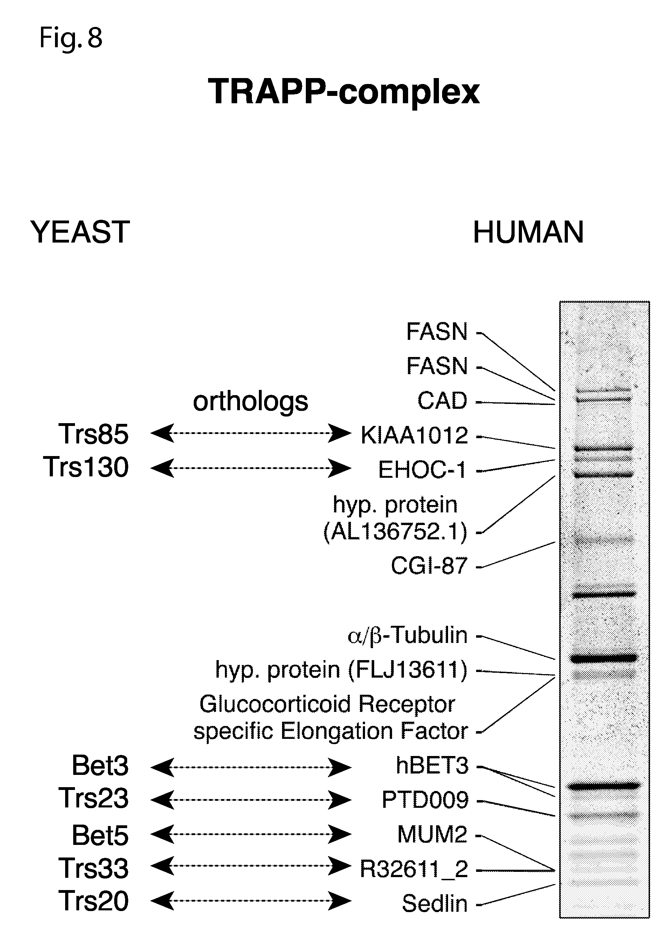

## Claims

1. A protein complex selected from complex (I) and comprising
(a) at least one first protein, which first protein is selected from the group of proteins in table 1, sixth column of a given complex, or a functionally active derivative thereof, or a functionally active fragment thereof, or a homologue thereof, or a variant of said protein, the variant being, the variant being encoded by a nucleic acid that hybridizes to the nucleic acid encoding said protein under low stringency conditions; and
(b) at least one second protein, which second protein is selected from the group of proteins in table 1, seventh column of said given complex, or a functionally active derivative thereof, or a functionally active fragment thereof, or a homologue thereof, or a variant of said second protein, said variant being encoded by a nucleic acid that hybridizes to the nucleic acid encoding said protein under low stringency conditions;
and a complex (II) comprising at least two of said second proteins,
wherein said low stringency conditions comprise hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

2. A protein complex comprising a first protein selected from the proteins listed in table 1, second column of a given complex or a homologue or variant thereof, or a functionally active fragment or functionally active derivative of said first protein, the variant being encoded by a nucleic acid that hybridizes to the nucleic acid of said first protein under low stringency conditions, and at least one second protein selected from the group of proteins in table 1, seventh column of a given complex, or a variant or homologue thereof, or a functionally active fragment or a functionally active derivative of said second protein, the variant of said second protein being encoded by a nucleic acid that hybridizes to the nucleic acid of said second protein under low-stringency conditions, and wherein said low stringency conditions comprise hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

3. A protein complex comprising all proteins selected from the proteins in table 1, third column of a given complex or at least one protein being a homologue thereof, or a variant thereof or functionally active fragment or functionally active derivative of said protein, said variant being encoded by a nucleic acid that hybridizes to the nucleic acid of said protein under low stringency conditions;
wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4), 5 mM EDTA, and 0.1% SDS for 1.5 hours at 60 °C.

4. A protein complex that comprises all proteins as listed in table 1, third column for a given complex or at least one protein being a homologue or a variant thereof, or a functionally active fragment or a functionally active derivative thereof, the variant being encoded by a nucleic acid that hybridizes to the nucleic acid of any of said proteins under low stringency conditions, except at least one protein of the proteins listed in table 1, third column, wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4), 5 mM EDTA, and 0.1% SDS for 1.5 hours at 60°C, with the provisio that the complex comprises at least one protein selected from table 1, seventh column of a given complex.

5. The complex of any of claims 1 - 4 comprising at least one functionally active derivative of said first protein and/or a functionally active derivative of said second protein, wherein the functionally active derivative is a fusion protein comprising said first protein or said second protein fused to an amino acid sequence different from the first protein or second protein.

6. The complex of claim 5 wherein the functionally active derivative is a fusion protein comprising said first protein or said second protein fused to an affinity tag or label.

7. The complex of any of claims 1 - 4 comprising a fragment of said first protein and/or a fragment of said second protein, which fragment binds to another protein component of said complex.

8. The complex of any of claims 1 - 7 that is involved in at least one biochemical activity as stated in table 2, column 8 for a given complex.

9. A process for preparing a complex of any of claims 1 - 8 and optionally the components thereof comprising the following steps:
expressing a protein of the complex, preferably a tagged protein, in a target cell, or a tissue or an organ, isolating the protein complex which is attached to the protein, preferably the tagged protein, and optionally disassociating the protein complex and isolating the individual complex members.

10. The process according to claim 9 wherein the tagged protein comprises two different tags which allow two separate affinity purification steps.

11. The process according to any of claims 9 - 10 wherein the two tags are separated by a cleavage site for a protease.

12. Component of a protein complex obtainable by a process according to any of claims 9-11.

13. Protein selected from the group of proteins in table 1, ninth column of a given complex or a homologue or a variant of thereof, or a functionally active fragment or a functionally active derivative of said protein, the variant being encoded by a nucleic acid that hybridizes to the nucleic acid encoding said protein under low stringency conditions, wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.

14. Nucleic acid encoding a protein according to claim 13.

15. Construct, preferably a vector construct, comprising
(a) a nucleic acid according to claim 14 and at least one further nucleic acid which is normally not associated with said nucleic acid, or
(b) at least two separate nucleic acid sequences each encoding a different protein, or a functionally active fragment or a functionally active derivative thereof, or a homologue or a variant thereof, at least one of said proteins being selected from the first group of proteins according to claim 1 (a) and at least one of said proteins, being selected from the second group of proteins according to claim 1 (b) or
(c) at least two separate nucleic acid sequences each encoding a different protein, or a functionally active fragment or a functionally active derivative thereof, or a homologue or a variant thereof, said proteins being selected from the proteins of complex (II) according to claim 1.

16. Host cell, containing a vector comprising at least one nucleic acid of claim 14 and/or a construct of claim 15 or containing several vectors each comprising at least one nucleic acid encoding at least one protein selected from the first group of proteins according to claim 1 (a) and at least one nucleic acid encoding at least one protein selected from the second group of proteins according to claim 1 (b).

17. An antibody or a fragment of said antibody containing the binding domain thereof, selected from an antibody or fragment thereof, which binds the complex of any of claims 1 - 8 and which does not bind any of the proteins of said complex when uncomplexed and an antibody or a fragment of said antibody containing the binding domain thereof which binds to any of the proteins of the group of proteins according to claim 13.

18. A kit comprising in one or more containers:
(a) the complex of any of claims 1 - 8 and/or the proteins of claim 13 and/or
(b) an antibody according to claim 17 and/or
(c) a nucleic acid encoding a protein of the complex of any of claims 1 - 8 and/or a protein of claim 13 and/or
(d) cells expressing the complex of any of claims 1 - 8 and/or a protein of claim 13 and, optionally,
(e) further components such as reagents, buffers and working instructions.

19. The kit according to claim 18 for processing a substrate of a complex of any one of claims 1 - 8.

20. The kit according to claim 18 for the diagnosis or prognosis of a disease or a disease risk, preferentially for a disease or disorder as stated in table 4, third column for a given complex..

21. Array, preferably a microarray, in which at least a complex according to any of claims 1 - 8 and/or at least one protein according to claim 13 and/or at least one antibody according to claim 17 is attached to a solid carrier.

22. A process for modifying a substrate of a complex of any one of claims 1 - 8 comprising the step of bringing into contact a complex of any of claims 1 - 8 with said substrate, such that said substrate is modified.

23. A pharmaceutical composition comprising the protein complex of any of claims 1 - 8 and/or a protein according to claim 13.

24. A pharmaceutical composition according to claim 23 for the treatment of diseases and disorders, preferentially for diseases or disorders as stated in table 4, third column of said complex.

25. A method for screening for a molecule that binds to a complex of any one of claims 1 - 8 and/or a protein of claim 13, comprising the following steps:
(a) exposing said complex or protein, or a cell or organism containing said complex or said protein, to one or more candidate molecules; and
(b) determining whether said candidate molecule is bound to the complex or protein.

26. A method for screening for a molecule that modulates directly or indirectly the function, activity, composition or formation of a complex of any one of claims 1 - 8 comprising the steps of:
(a) exposing said complex, or a cell or organism containing said complex to one or more candidate molecules; and
(b) determining the amount of, activity of, protein components of, and/or intracellular localization of, said complex and/or the transcription level of a gene regulated by the complex and/or the abundance and/or activity of a protein or protein complex dependent upon the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene regulated by the complex and/or the abundance and/or activity of a protein or protein complex dependent on the function of the complex and/or product of a gene dependent on the complex in the absence of said candidate molecules indicates that the molecule modulates function, activity, or composition of said complex.

27. The method of claim 26, wherein the amount of said complex is determined.

28. The method of claim 26, wherein the activity of said complex is determined.

29. The method of claim 28, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.

30. The method of claim 26, wherein the amount of the individual protein components of said complex is determined.

31. The method of claim 30, wherein said determining step comprises determining whether any of the proteins listed in table 1, third column of said complex, or a functionally active fragment or a functionally active derivative thereof, or a variant or a homologue thereof, the variant being encoded by a nucleic acid that hybridizes to the nucleic acid of said protein under low-stringency conditions, is present in the complex.

32. The method of any of claims 26 - 31, wherein said method is a method of screening for a drug for treatment or prevention of a disease or disorder, preferentially of a disease or disorder selected from the diseases or disorders as listed in table 4, third column.

33. Use of a molecule that modulates the amount of, activity of, or the protein components of the complex of any one of claims 1 - 8 for the manufacture of a medicament for the treatment or prevention of a disease or disorder, preferentially of a disease or disorder as listed in table 4, third column.

34. A method for the production of a pharmaceutical composition comprising carrying out the method of claims 26 - 31 to identify a molecule that modulates the function, activity, composition or formation of said complex, and further comprising mixing the identified molecule with a pharmaceutically acceptable carrier.

35. A method for diagnosing or screening for the presence of a disease or disorder or a predisposition for developing a disease or disorder in a subject, which disease or disorder is **characterized by** an aberrant amount of, component disposition of, or intracellular localization of the complex of any one of the claims 1 - 8, comprising determining the amount of, activity of, protein components of, and/or intracellular localization of, said complex and/or the transcription level of a gene regulated by the complex and/or the abundance and/or activity of a protein or protein complex dependent on the function of the complex and/or product of a gene dependent on the complex in a comparative sample derived from a subject, wherein a difference in said amount, activity, or protein components of, said complex in a corresponding sample from a subject not having the disease or disorder or predisposition indicated the presence in the subject of the disease or disorder or predisposition in the subject.

36. The method of claim 35, wherein the amount of said complex is determined.

37. The method of claim 35, wherein the activity of said complex is determined.

38. The method of claim 37, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.

39. The method of claim 35, wherein the amount of the individual protein components of said complex is determined.

40. The method of claim 39, wherein said determining step comprises determining whether any of the proteins according to claim 13 is present in the complex.

41. The complex of any one of claims 1 - 8, or a protein of claim 13 or an antibody or fragment thereof of claim 17, for use in a method of diagnosing a disease or disorder, preferentially of a disease or disorder as listed in table 4, third column of said complex.

42. A method for treating or preventing a disease or disorder **characterized by** an aberrant amount of, activity of, component composition of or intracellular localization of, the complex of any one of claims 1 - 8, comprising administering to a subject in need of such treatment or prevention a therapeutically effective amount of one or more molecules that modulate the amount of, activity of, or protein composition of, said complex.

43. The method according to claim 42, wherein said disease or disorder involves decreased levels of the amount or activity of said complex.

44. The method according to claim 42, wherein said disease or disorder involves increased levels of the amount or activity of said complex.

45. Complex of claims 1 - 8 and/or a protein as listed in table 1, seventh column of said complex as a target for an active agent of a pharmaceutical, preferably a drug target, in the treatment or prevention of a disease or disorder, preferentially of a disease or disorder as listed in table 4, third column of said complex.
